Europäisches Patentamt

European Patent Office

Office européen des brevets

(11) Veröffentlichungsnummer: **0 001 777**
**B1**

(12) **EUROPÄISCHE PATENTSCHRIFT**

(21) Anmeldenummer: **78101151.5**

(22) Anmeldetag: **14.10.78**

(51) Int. Cl³: **C 07 C  68/00,**
**C 07 C  69/96**

(54) Verfahren zur Herstellung von Dialkylcarbonaten

(30) Priorität: **29.10.77 DE 2748718**

(43) Veröffentlichungstag der Anmeldung:
**16.05.79 Patentblatt 79/10**

(45) Bekanntmachung des Hinweises auf die Patenterteilung:
**23.07.80 Patentblatt 80/15**

(84) Benannte Vertragsstaaten:
**BE CH DE FR GB LU NL**

(56) Entgegenhaltungen:
**DE - A - 2 555 567**
**US - A - 3 248 415**
**US - A - 3 642 858**

(73) Patentinhaber: **Bayer AG**
**Zentralbereich Patente, Marken und Lizenzen**
**D - 5090 Leverkusen 1, Bayerwerk (DE)**

(72) Erfinder: **Buysch, Hans-Josef, Dr.**
**Brandenburger Strasse 28**
**D - 4150 Krefeld (DE)**
**Krimm, Heinrich, Dr.**
**Heyenbaumstrasse 65**
**D - 4150 Krefeld (DE)**
**Rudolph, Hans, Dr.**
**Haydnstrasse 9**
**D - 4150 Krefeld (DE)**

Courier Press, Leamington Spa, England.

Verfahren zur Herstellung von Dialkylcarbonaten

Die Erfindung betrifft ein Verfahren zur Herstellung von Dialkylcarbonaten durch katalytische Umsetzung von Alkylenoxiden mit Alkoholen und Kohlendioxid.

Es ist überraschend, daß sich Dialkylcarbonate durch Umsetzung von Alkylenoxiden mit Alkoholen und Kohlendioxid überhaupt herstellen lassen.

Es war vielmehr zu erwarten, daß bei der Umsetzung von Alkylenoxiden mit Alkoholen und Kohlendioxid Polymerisations-reaktionen stattfinden, die z.B. zu Polycarbonaten und Polyglykolen führen.

Aus der US—PS 3 248 415 und der US—PS 3 248 416 its nämlich bekannt, daß sich Alkylenoxide und Kohlendioxid bzw. Alkylenoxide, Kohlendioxid und Glykolcarbonate in Gegenwart geringer Mengen Di- oder Polyglykole unter dem katalytischen Einfluß von Basen zu Polycarbonaten umsetzen, die allerdings überwiegend Polyalkylenoxideinheiten enthalten und daher im strengen Sinne als Polyätherpolycarbonate anzusprechen sind.

Weiterhin ist aus Makromol. Chem. *130*, 210 (1969) bekannt, daß bei der Reaktion von Alkylenoxiden mit Kohlendioxid in Gegenwart von Zinkdiäthyl als Katalysator neben geringen Mengen an alternierenden Copolymerisaten von Alkylenoxiden mit Kohlendioxid große Mengen an Polyglykolen erhalten werden.

Neben den Polymerisationsreaktionen war die Bildung von Alkylglykoläthern als Nebenreaktion zu erwarten. Aufgrund der großen Zahl an vorhandenen Hydroxygruppen — es werden dem Alkylenoxid äquivalente Mengen an Alkoholen zugesetzt — sollte das Alkylenoxid mit dem Alkohol in beträchtlichem Maße zu Alkylglykoläthern abreagieren.

Es wurde nun gefunden, daß man Dialkylcarbonate in guten Ausbeuten herstellen kann, wenn man Alkylenoxide mit 2 bis 8 Kohlenstoffatomen mit aliphatischen und/oder cycloaliphatischen Alkoholen mit 1 bis 18 Kohlenstoffatomen und Kohlendioxid in Gegenwart von Katalysatoren bei Temperaturen im Bereich von 90 bis 280°C umsetzt, wobei das Molverhältnis von Alkohol zu Alkylenoxid 1 : 1 bis 1 : 20 beträgt und mit einem Überschuß an Kohlendioxid bei einem Kohlendioxidpartialdruck von 0,5 bis 500 bar gearbeitet wird.

Als aliphatische und/oder cycloaliphatische Alkohole kommen solche mit 1 bis 18, bevorzugt 1 bis 12, Kohlenstoffatomen in Frage.

Beispielsweise seien genannt: Methanol, Äthanol, Propanol, Isopropanol, n-Butanol, Isobutanol, Amylalkohol, Cyclohexanol, Octanol, Decanol und Cyclododecanol.

Als Alkylenoxide werden solche mit 2 bis 8, bevorzugt mit 2 bis 3, Kohlenstoffatomen in das erfindungsgemäße Verfahren eingesetzt. Z.B. seien genannt:

Vinyloxiran, Epichlorhydrin, Butylenoxid-1.2 bzw. 2.3, Cyclohexenoxid, Äthylenoxid und Propylenoxid, bevorzugt werden Äthylenoxid und Propylenoxid eingesetzt.

Als Katalysatoren für das erfindungsgemäße Verfahren eignen sich basische organische Stickstoffverbindungen, insbesondere tert. Amine mit 3 bis 18, bevorzugt mit 3 bis 12, Kohlenstoffatomen, Beispielsweise seien genannt: Triäthylamin, Tributylamin, Triäthanolamin, Dimethylbenzylamin und Dimethylstearylamin.

Weiterhin sind heterocyclische aromatische Stickstoffverbindungen mit 2 bis 15, bevorzugt mit 2 bis 12, Kohlenstoffatomen geeignet. Genannt seien z.B. Pyridin, Pyrimidin, Imidazol, Triazol, Tetrazol, Benzimidazol, Benztriazol und Benzthiazol.

Als Katalysatoren lassen sich auch Hydrazine, wie Hydrazin selbst, Dimethylhydrazin und Dibenzylhydrazin sowie Amidine, wie Formamidin, Acetamidin, cyclische Amindine, wie 2-Alkyl-imidazoline-2, Guanidin und Harnstoff verwenden. Daneben können die Salze aller genannten Stickstoffverbindungen mit schwachen und starken Säuren, wie Carbonsäuren mit 1 bis zu 18, Kohlenstoffatomen, Kohlensäure oder Halogenwasserstoffsäuren sowie Quaternierungsprodukte der genannten Stickstoffverbindungen, die z.B. durch Umsetzung der Stickstoffverbindungen mit Alkylierungsmitteln z.B. Alkylhalogeniden gewonnen werden, als Katalysatoren eingesetzt werden. Solche Verbindungen sind z.B. Tetraäthylammoniumbromid oder -jodid, Tetrabutylammoniumchlorid, Dimethylpiperidiniumjodid, N-Methyl-pyridiniumbromid, N.N'-Dimethylimidazoliumchlorid und N-Methyl-benzthiazoliumjodid.

Auch andere salzartige Verbindungen, wie die Sulfonium- und Phosphoniumsalze von Halogenwasserstoffsäuren, z.B. Triäthylsulfoniumjodid, Tetraäthylphosphoniumbromid, Tetrabutylphosphoniumjodid und Triphenylbenzyl-phosphoniumchlorid sind als Katalysatoren geeignet.

Eine weitere Klasse katalytisch wirksamer Substanzen sind die Oxide, Hydroxide und Salze der Alkali- und Erdalkalimetalle, wie LiOH, LiCl, $Li_2CO_3$, NaOH, NaOAc, $K_2CO_3$, NaJ, KBr, K-stearat, Sr-stearat, $CaCl_2$, $CaJ_2$ ferner die Oxide, Hydroxide, Carbonate und Salze der Halogenwasserstoffsäuren von Schwermetallen wie $ZnCl_2$, $CdJ_2$, $CoBr_2$, $FeCl_3$, $NiCl_2$, $MnJ_2$, $CoCo_3$, $PbCl_2$, sodann Verbindungen des Zinns, vor allem auch mit organischen Resten mit bis zu 46 Kohlenstoffatomen, wie $SnCl_2$, $(But)_2SnO$, $(But)_2Sn(Laurat)_2$, $(But)_2Sn(OCH_3)_2$, $Na_2SnO_2$, $Na_2SnO_3$ und schließlich die Oxide, Hydroxide und Salze des Thalliums mit anorganischen und organischen Säuren wie $Tl_2CO_3$, $Tl_2O$, TlOAc, $TlNO_3$, $Tl(OAc)_3$, $Tl_2O_3$ und TlJ. In manchen Fällen erweist es sich als günstig, Kombination der genannten Katalysatoren wie $NaJ/Tl_2CO_3$, $NaJ/Li_2CO_3$, $CoCO_3/TlJ$ oder N-Methyl-pyridiniumbromid/Guanidincarbonat zu verwenden, oder aber den Katalysator erst im Reaktionsgemisch zu erzeugen, z.B. durch Einsetzen von Triäthylamin und Äthyljodid.

Die Katalysatoren können auch auf feste Träger aufgebracht werden, z.B. auf MgO und $Al_2O_3$, oder können in Form von Festkatalysatoren verwendet werden, z.B. als mit Divinylbenzol vernetzten Polystyrolen die Amid- oder quaternäre Ammoniumgruppen besitzen.

Bevorzugt verwendet als Katalysatoren werden die Halogenide, Alkoholate, Oxide, Hydroxide und Carbonate der Alkalimetalle und des Thalliums und die Halogenide der quartären Ammoniumbasen, insbesondere die Bromide und Jodide. Sehr vorteilhaft ist die Verwendung von Gemischen dieser Verbindungen, insbesondere solchen, die Thalliumverbindungen enthalten.

Das Molverhältnis von Alkohol zu Alkylenoxid ist beim erfindungsgemäßen Verfahren nicht kritisch. Es liegt normalerweise im Bereich von etwa 1:1 bis 1:20, bevorzugt 1:2 bis 1:15. Das Molverhältnis kann jedoch auch außerhalb des angegebenen Bereiches liegen. Ein Überschuß an Alkohol wird im allgemeinen bevorzugt eingesetzt, um das Reaktionsgleichgewicht in Richtung auf die gewünschten Dialkylcarbonate zu verschieben.

Kohlendioxid wird üblicherweise im Überschuß von 0,1 bis 100 Mol/Mol Äthylenoxid eingesetzt. Der Kohlendioxidpartialdruck sollte dabei im Bereich von etwa 0,1 bis 1000 bar, vorzugsweise 0,5 bis 500 bar, besonders bevorzugt bei 1 bis 200 bar, liegen.

Die Reaktionstemperaturen des erfindungsgemäßen Verfahrens betragen im allgemeinen etwa 70 bis 300°C, vorzugsweise 90 bis 280°C, besonders bevorzugt 100 bis 250°C.

Die Reaktion kann diskontinuierlich oder kontinuierlich durchgeführt werden, beispielsweise in einem Rohrreaktor. Die Trennung der Reaktionskomponenten geschieht nach üblichen Methoden, beispielsweise durch fraktionierte Destillation. Der im Rückstand verbleibende Katalysator kann wieder verwendet werden.

Nach dem erfindungsgemäßen Verfahren können die Dialkylcarbonate in Ausbeuten von über 97 % d. Th. erhalten werden.

Die Produkte des erfindungsgemäßen Verfahrens sind geeignet als Lösungsmittel für Cellulosederivate und als Ausgangsmaterialien für die Herstellung von Diarylcarbonaten, aliphatischen und aromatischen Polycarbonaten, Arznei- und Pflanzenschutzmittel (DT—OS 2 528 412, DT—AS 1 031 512, US—PS 3 933 846, J. Amer. Chem. Soc. *52*, 314 (1930), Ullman's Encyclopädie der technischen Chemie, 3. Auflage, Bd. 9, S. 776 ff (1957)).

Die folgenden Beispiele sollen das erfindungsgemäße Verfahren näher erläutern, ohne es jedoch auf diese Beispiele einzuschränken.

## BEISPIEL 1

Ein Gemisch aus 640 g (20 Mol) Methanol und 44 g (1 Mol) Äthylenoxid wird in Gegenwart von 1 g Natriumjodid und 0.2 g Thalliumcarbonat unter einem $CO_2$-Druck von 70 bar 2 h bei 120°C gehalten. Nach Abkühlen wird entspannt.

Das Gaschromatogramm des Reaktionsgemisches zeigt, daß ca. 7 Gew.-% an Dimethylcarbonat im Reaktionsgemisch vorliegen. Der Gehalt an Diglykol beträgt ce. 0.3, an Triglykol 0,1 und an Methylglykol 0.5 Gew.-%.

Die destillative Aufarbeitung des Reaktionsgemischens ergibt neben der 51 g Dimethylcarbonat enthaltenden Methanolfraktion 49 g einer bei 60—80°C/0,8 torr siedenden aus ca. 30 g Glykol und 19 g Glykolcarbonat bestehenden Fraktion. Der Rückstand beträgt 1,8 g und entspricht praktisch den eingesetzten Katalysatormengen.

## BEISPIEL 2

Beispiel 1 wird wiederholt Im Anschluß an die Reaktion (2 h bei 150°C unter einem Druck von 88 bar $CO_2$) läßt man das $CO_2$ nach Abkühlen entweichen und hält die Temperatur weitere 2 h auf 150°C.

Laut Gaschromatogramm enthält das Reaktionsgemisch 0,1 % Di- und Triglykol und ca 0,7 % Methylglykol. Nach Aufarbeitung, wie in Beispiel 7, erhält man eine Methanolfraktion bestehend aus 85 g Dimethylcarbonat und eine Glykolfraktion, die 3—4 g Glykolcarbonat enthält. Der Destillationsrückstand beträgt 2,5 g.

Das eingesetzt Äthylenoxid ist also zu über 94 % in Glykol und ein äquivalenter Anteil an Methanol und $CO_2$ in Dimethylcarbonat übergegangen. Das gebildete Glykolcarbonat läßt sich durch Umesterung in Dimethylcarbonat überführen, so daß dann die Gesamtausbeute an Dimethylcarbonat bezogen auf eingesetztes Äthylenoxid >97 %. d. Th. beträgt.

## BEISPIEL 3

Ein Gemisch aus 640 g (20 Mol) Methanol, 53 g (1,2 Mol) Äthylenoxid, 2 g Natriumjodid und 0,2 g Thalliumhydroxid wird unter 100 bar $CO_2$ 2 h bei 160°C gehalten, $CO_2$ abgeblasen und noch 1/2 h bei 150°C gehalten. Nach destillativer Aufarbeitung erhält man eine Methanolfraktion, die 102 g Dimethylcarbonat ,und ca. 1 g Methylglykol enthält. Der Destillationsrückstand beträgt 3 g und entspricht damit praktisch der katalysatormenge.

## BEISPIEL 4

Ein Gemisch wie in Beispiel 1, das als Katalysator 1 g Natriumjodid und kein Thalliumsalz enthält, wird 2 h bei 150°C umgesetzt. Die destillative abgetrennte Methanolfraktion enthält 57 g Dimethylcarbonat und 0,2 g Methylglykol. Als Destillationsrückstand verbleibt mit 1 g das eingesetzte Natriumjodid. Äthylenoxid wird nicht quantitativ umgesetzt.

### BEISPIEL 5

Ein Gemisch wie in Beispiel 1 wird in Gegenwart von 1 g Lithiumhydroxid bei 50 bar $CO_2$-Druck 2 h bei 150°C umgesetzt. Man erhält bei unvollständigem Äthylenoxidumsatz nach Aufarbeitung 45 g Dimethylcarbonat und 0,3 g Methylglykol in der Methanolfraktion. Der Rückstand beträgt 1,5 g.

### BEISPIEL 6

Ein Gemisch wie in Beispiel 1 wird in Gegenwart von 1 g Triäthanolamin als Katalysator 2 h bei 150°C und 30 bar $CO_2$-Druck reagieren lassen. Man erhält bei unvollständigem Äthylenoxidumsatz 33 g Dimethylcarbonat und 0,5 g Methylglykol in der Methanolfraktion. Der Rückstand beläuft sich auf 2,5 g.

### BEISPIEL 7

Ein Gemisch wie in Beispiel 1 wird in Gegenwart von 0,2 g Natriumcarbonat als Katalysator bei 80 bar $CO_2$-Druck 2 h auf 150°C erhitzt. Die abgetrennte Methanolfraktion enthält 23 g Dimethylcarbonat und 0,5 g Methylglykol. Als Rückstand verbleiben 1,5 g.

### BEISPIEL 8

Ein Gemisch wie in Beispiel 1 wird in Gegenwart von 0,1 g Natriumjodid als Katalysator unter einem $CO_2$-Druck von 80 bar 2 h auf 150°C erhitzt. Man erhält 19 g Dimethylcarbonat und 0,6 g Methylglykol in der Methanolfraktion.

### BEISPIEL 9

Ein Gemisch wie in Beispiel 1 wird in Gegenwart von 1 g Imidazol als Katalysator bei einem $CO_2$-Druck von 20—30 bar 2 h auf 150°C erhitzt. Die Ausbeute an Dimethylcarbonat beträgt 18 g.

### BEISPIEL 10

Ein Gemisch wie in Beispiel 1 wird in Gegenwart von 1 g 1,5-Diazabicyclo-(0.4.5)-undecen-5 als Katalysator bei 80 bar $CO_2$-Druck 2 h auf 150°C erhitzt. Die Ausbeute an Dimethylcarbonat beträgt 16 g.

### BEISPIEL 11

Beispiel 1 wird wiederholt mit 0,5 g Tetraäthylammoniumbromid als Katalysator bei 150°C. Die Ausbeute an Dimethylcarbonat beträgt 25 g.

### BEISPIEL 12

Wird Beispiel 10 wiederholt mit 0,5 g Tetrabutylammoniumjodid als Katalysator, so erhält man 28 g Dimethylcarbonat.

### BEISPIEL 13

Ein Gemisch von 640 g (20 Mol) Methanol und 132 g (3 Mol) Äthylenoxid wird in Gegenwart von 1 g Natriumjodid und 0,2 g Thalliumhydroxid bei einem $CO_2$-Druck von 110 bar 2 h bei 150°C gehalten. Bei unvollständigem Umsatz an Äthylenoxid erhält man 123 g Dimethylcarbonat und 0,6 g Methylglykol in der Methanolfraktion. Als Rückstand verbleiben 2 g.

### BEISPIEL 14

Beispiel 1 wird bei 150°C wiederholt mit 1 g Lithiummethylat als Katalysator. Die Ausbeute an Dimethylcarbonat beträgt 53 g.

### BEISPIEL 15

Beispiel 1 wird wiederholt mit 1 g Guanidincarbonat als Katalysator. Die Ausbeute an Dimethylcarbonat beträgt 11 g.

### BEISPIEL 16

Beispiel 1 wird wiederholt mit 1 g Lithiumjodid als Katalysator. Die Ausbeute an Dimethylcarbonat beträgt 56 g.

### BEISPIEL 17

Beispiel 1 wird wiederholt bei 70 bar $CO_2$-Druck und 120°C. Die Ausbeute an Dimethylcarbonat beträgt 78 g.

### BEISPIEL 18

Ein Gemisch von 796 g (17,3 Mol) Äthanol und 36 g (0,82 Mol) Äthylenoxid wird unter den Bedingungen von Beispiel 1 miteinander umgesetzt.

Die Ausbeute an Diäthylcarbonat beträgt 22 g entsprechend einem Umsatz von 23 % des eingesetzten Äthylenoxids. Äthylglykol wird nicht gefunden. Der Destillationsrückstand beträgt 2 g und entspricht damit der Menge an eingesetztem Katalysator.

### BEISPIEL 19

Wiederholt man Beispiel 18 mit 1 g Imidazol statt des Katalysators aus Beispiel 1 und hält 2 h bei 200°C unter 88 bar $CO_2$-Druck, so erhält man eine Menge Diäthylcarbonat, die einem Umsatz des eingesetzten Äthylenoxid von 43 % entspricht.

### BEISPIEL 20

Ein Gemisch von 749 g (16,3 Mol) Äthanol und 45 g (0,775 Mol) Propylenoxid wird unter einem $CO_2$-Druck von 80 bar und in Gegenwart von 0,18 g Thalliumcarbonat und 1,8 g Natriumjodid 2 h bei 150°C gehalten. Nach Abblasen von $CO_2$ wird noch 2 h bei 200°C gehalten. Die Ausbeute an Diäthylcarbonat beträgt 76 g entsprechend einem Umsatz an Propylenoxid von 83 %. Propylenglykolmonoäthyläther ist nicht gebildet worden. Die Rückstandsmenge der Destillation entspricht der des eingesetzten Katalysators.

### BEISPIEL 21

Beispiel 1 wird wiederholt, wobei 88 g (2 Mol) Kohlendioxid, entsprechend einem Molverhältnis von Kohlendioxid zu Äthylenoxid wie 2:1 eingesetzt wird. Nach 2 Stunden bei 150°C erhält man 65 g Dimethylcarbonat.

### BEISPIEL 22

Wiederholt man Beispiel 21 mit 52 g (1,2 Mol) Kohlendioxid, entsprechend einem Molverhältnis von Kohlendioxid zu Äthylenoxid wie 1,2:1, so findet man 68 g Dimethylcarbonat und 2 g Destillationsrückstand. Polyglykole werden demnach nicht gebildet.

### Patentansprüche

1. Verfahren zur Herstellung von Dialkylcarbonaten, dadurch gekennzeichnet, daß man Alkylenoxide mit 2 bis 8 Kohlenstoffatomen mit aliphatischen und/oder cycloaliphatischen Alkoholen mit 1 bis 18 Kohlenstoffatomen und Kohlendioxid in Gegenwart von Katalysatoren bei Temperaturen im Bereich von 90 bis 280°C umsetzt, wobei das Molverhältnis von Alkohol zu Alkylenoxid 1:1 bis 1:20 beträgt und mit einem Überschuß an Kohlendioxid bei einem Kohlendioxidpartialdruck von 0,5 bis 500 bar gearbeitet wird.

2. Verfahren nach Anspruch 1, dadurch gekennzeichnet, daß man bei Temperaturen im Bereich von 100 bis 250°C arbeitet.

3. Verfahren nach Anspruch 1, dadurch gekennzeichnet, daß man mit einem Überschuß an Kohlendioxid bei einem Kohlendioxidpartialdruck von 1 bis 200 bar arbeitet.

4. Verfahren nach Anspruch 1, dadurch gekennzeichnet, daß man als Alkylenoxide Ethylenoxid und Propylenoxid verwendet.

### Revendications

1. Procédé de préparation de carbonates de dialkyle, caractérisé en ce que l'on fait réagir des oxydes d'alkylène en C$_2$—C$_8$ avec des alcools aliphatiques ou cycloaliphatiques en C$_1$—C$_{18}$ ou leurs mélanges et le dioxyde de carbone en présence de catalyseurs, à des températures de 90 à 280°C, avec un rapport molaire alcool/oxyde d'alkylène de 1:1 à 1:20 et en opérant avec un excès de dioxyde de carbone sous une pression partielle de dioxyde de carbone de 0,5 à 500 bars.

2. Procédé selon la revendication 1, caractérisé en ce que l'on opère à des températures de 100 à 250°C.

3. Procédé selon la revendication 1, caractérisé en ce que l'on opère avec un excès de dioxyde de carbone sous une pression partielle de dioxyde de carbone de 1 à 200 bars.

4. Procédé selon la revendication 1, caractérisé en ce que l'on utilise comme oxydes d'alkylène l'oxyde d'éthylène et l'oxyde de propylène.

### Claims

1. Process for the preparation of dialkyl carbonates, characterised in that alkylene oxides having 2 to 8 carbon atoms are reacted with aliphatic and/or cycloaliphatic alcohols having 1 to 18 carbon atoms and carbon dioxide in the presence of catalysts at temperatures in the range from 90 to 280°C; the molar ratio of alcohol to alkylene oxide is 1:1 to 1:20 and the reaction is carried out with an excess of carbon dioxide under a partial pressure of carbon dioxide of 0.5 to 500 bars.

2. Process according to Claim 1, characterised in that the reaction is carried out at temperatures in the range from 100 to 250°C.

3. Process according to Claim 1, characterised in that the reaction is carried out with an excess of carbon dioxide under a partial pressure of carbon dioxide of 1 to 200 bars.

4. Process according to Claim 1, characterised in that ethylene oxide and propylene oxide are used as the alkylene oxides.